# EUROPEAN PATENT SPECIFICATION

(11) **EP 3 629 045 B1**
(45) Date of publication and mention of the grant of the patent: **13.01.2021**
(21) Application number: 18197218.3
(22) Date of filing: 27.09.2018
(51) Int. Cl.: G01R 33/28, A61M 25/02, A61M 25/06, A61M 25/01

(54) **VASCULAR ACCESS DEVICE AND METHOD FOR TRACKING A MEDICAL INSTRUMENT IN A BODY REGION OF A PATIENT**
VORRICHTUNG FÜR VASKULÄREN ZUGRIFF UND VERFAHREN ZUR VERFOLGUNG EINES MEDIZINISCHEN INSTRUMENTS IN EINER KÖRPERREGION EINES PATIENTEN
DISPOSITIF D'ACCÈS VASCULAIRE ET PROCÉDÉ DE SUIVI D'UN INSTRUMENT MÉDICAL DANS UNE ZONE CORPORELLE D'UN PATIENT

(43) Date of publication of application: 01.04.2020
(73) Proprietor: Siemens Healthcare GmbH, 91052 Erlangen (DE)
(72) Inventor: Hengerer, Arne, 91096 Möhrendorf (DE); Matschl, Volker, 96049 Bamberg (DE)

(56) References cited:
- WO-A1-2017/196734
- US-A1- 2003 050 662
- US-A1- 2009 234 445
- US-A1- 2014 228 905

## Description

The invention concerns a vascular access device, comprising a vascular cannula having a catheter portion for insertion into a blood vessel, in particular a vein, of a patient and a fixing portion for fixing the vascular cannula to a skin of a patient. The invention further concerns a method for tracking a medical instrument in a body region of a patient.

In medicine, for many therapies or examinations, access to a blood vessel of a patient, in most cases a vein, is required. In this regard, vascular access devices (VAD), sometimes also called venous access devices, are used. Such vascular access devices usually comprise a vascular cannula having a catheter portion that is inserted into the blood vessel. The vascular cannula also usually has a fixing portion, in particular comprising wings, which allow to fix the vascular cannula to the skin of the patient. To this end, adhesives and/or plasters may be used. The vascular cannula may further comprise a valve to allow injection of medications, for example drugs, using a syringe and/or an end which allows connection to an intravenous infusion line. Often, additionally a so-called mandrin is provided, which is removed after successful positioning of the vascular cannula. Mandrins are usually made of metal and positioned on the inside of the vascular cannula, in particular the catheter portion.

To place vascular access, usually, the insertion region is first treated with a local skin antiseptic. After this, the insertion region may be covered using a surgical incise drape. Then, the actual insertion takes place using a local anaesthetic. The blood vessel is punctured using the vascular cannula. The insertion into the blood vessel can be seen from blood filling occurring in the back part of the cannula.

While the so-created vascular access may be used for medication, it is often also used as an entry point for a medical instrument, for example, a guide wire for another instrument, for example a therapeutic catheter, or the like. After the mandrin is removed, the medical instrument is inserted into the blood vessel via the vascular cannula, which is often done using an image modality for tracking, for example angiography. In the case of such a minimally invasive intervention, if the medical instrument is a guide wire, the guide wire can now be fixed and the vascular cannula can be removed. While doing this, the blood vessel has to be compressed at the access point to fix the position of the guide wire. After this, a sluice or already the therapeutic or treatment catheter is slid over the guide wire to its target position. Finally, the guide wire can be removed.

For such minimally invasive interventions on a patient, for which often the vena cava is used as vascular access point, in particular central venous catheters are used, in the case that a peripheral vein is used to establish access, a peripheral venous catheter may be used. For tracking the insertion of the vascular cannula or later on the insertion of the medical instrument through the venous cannula, as already mentioned, the progress may be tracked using an imaging modality, for example x-ray angiography. However, if an intervention is to be guided by magnetic resonance as the imaging modality, in most cases, magnetic resonance imaging does not provide enough local information to allow using this mobility for tracking at the access site. In this case, the venous access can be established and the medical instrument can be introduced without image guidance, however, it has been proposed to add a second imaging modality, for example ultrasound imaging or x-ray imaging.

See e.g. WO2017/196734 A1, US2009/234445 A1 or US2003/050662 A1.

It is an object of the current invention to provide a vascular access device and a method using this device allowing better guidance and tracking in magnetic resonance-guided interventions and/or procedures.

This object is achieved by providing a vascular access device according to claim 1 and a method according to claim 13. Advantageous embodiments are described by the dependent claims.

According to the invention, in a vascular access device as initially described, the fixing portion and/or a plaster used to fix the fixing portion to the skin comprises at least one local reception coil element for receiving magnetic resonance signals, in particular during placement of the vascular cannula and/or placement of an instrument through the cannula.

In a preferred embodiment of the invention, a local reception coil element is thus provided on the fixing portion, wherein, in particular, the fixing portion may comprise at least one wing on, or integrated into which the local reception coil element is positioned. Alternatively, but less preferred, a plaster with which the wing is to be fixed to the skin of the patient may have the local reception coil element attached to it or integrated into it.

Generally, the local reception coil element may be added in an inexpensive way. In particular, it is preferred that the local reception coil element is printed onto the plaster and/or the fixing portion, in particular the wing, or that the local reception coil element is provided on a flexible circuit board attached to the plaster and/or the fixing portion, in particular the wing. In this respect, it is also preferred that the local reception coil element, in particular the whole vascular access device, is configured for one-time use. This means that the vascular cannula, the plaster and/or its local reception coil element does not have to be sterilized. As already said, since the local reception coil element can be produced very cost-effective, that is, for a reasonable prize, a one-time use is possible also from a financial point of view. As known, the vascular cannula may be made from plastic or the like.

In summary, while the vascular access device and the radio frequency coils are, according to the state of the art, two independent systems, wherein usually a local coil is positioned first, whereafter sterility at the access site is ensured and vascular access is established. The current invention combines the access aid and the local reception coil in a manner that multiple advantages are obtained.

First of all, two steps in the workflow are combined, namely placement of the local coil and establishing the vascular access. Additionally, an optimal position of the access site and the reception coil is ensured. In particular, if the local reception coil element is fixed to the fixing portion, accidental movement of the local reception coil element is prevented.

In this case, a low-cost local reception coil element suffices to allow adequate image quality for the specific application. In particular if the local reception coil element is configured for one-time use, the local reception coil can be understood as an expendable item, which can be distributed using existing distribution channels, in particular by the vascular access device industry.

Providing the local reception coil as a part of the vascular access device, the local reception coil element is placed exactly where it needs to be placed to allow better image quality, in particular better signal-to-noise-ratio (SNR) in an imaging area covering the penetration depth and the penetrated blood vessel, in particular a vein, for magnetic resonance imaging. That is, the local reception coil element, which is, of course, a local reception coil element for magnetic resonance imaging, is configured to receive magnetic resonance signals from a body region, in particular comprising the access site and the vessel into which the vascular cannula is inserted, and to directly or indirectly transfer these received magnetic resonance signals to a magnetic resonance imaging device in which the patient is placed, in particular for an intervention or other procedure requiring vascular access.

In the scope of the current invention, it is in principle conceivable, that the vascular access device further comprises at least one connecting device, in particular having a cable, for the local reception coil element and/or that the local reception coil element comprises a pre-amplifier and/or at least one adaptation and/or detuning circuit. The at least one local reception coil element may be a conventional magnetic resonance local coil having, in particular, a detuning circuit and/or an adaptation circuit and/or a pre-amplifier, such that control and signal transmission may take place via a connecting device, in particular a coil plug. It may also be possible to use the local reception coil element also for transmitting magnetic resonance signals regarding excitation. However, regarding the cost aspect, but also regarding optional sterilizability, this configuration is less preferred.

In a preferred embodiment of the invention, however, the local reception coil element is configured to inductively couple to an external reception coil, in particular a body coil and/or an external local coil of the magnetic resonance imaging device. That is, a, so to say, cable-free local reception coil element is provided. The local reception coil element is inductively coupled to a body coil of the magnetic resonance imaging device and/or a local coil of the magnetic resonance imaging device.

Preferably, to achieve the inductive coupling to the external reception coil, the local reception coil element may comprise a self-resonant conducting structure and/or circuit. That is, a self-resonant structure can be provided on the substrate of the local reception coil element, or, in an alternative realization, a self-resonant circuit can be formed using discrete electronic components, for example SMD capacitors. Self-resonance can, for example, be achieved by providing overlapping conductor areas, which couple capacitively. The conductors themselves act as inductivities.

When using a self-resonant local reception coil element, the local reception coil element should be detuned during transmission of the excitation signals by corresponding transmitter coils of the magnetic resonance imaging device, that is, during excitation of spins to be imaged using a highfrequency field (B1 field). Thus, in an especially preferred embodiment, the local reception coil element further comprises at least one detuning element for detuning the local reception coil element during a magnetic resonance transmitting phase. In this manner, an excessive increase of the B1 field and thus an endangerment of the patient are prevented.

In concrete embodiments, the at least one detuning element may comprise at least one switching element, in particular a diode, the switching element closing on induction of a predefined voltage into the local reception coil element. Preferably, the diodes can be PIN diodes. In advantageous embodiments, two anti-parallel diodes may be used such that either when one of the diodes allows current to pass an additional detuning capacitor or inductance is added or both diodes allowing current to pass intrinsically decouple two then-formed sub-loops of the local reception coil element's main loop. Two anti-parallel PIN diodes may, for example, be used in the middle of the resonance loop of the local reception coil element, such that the two anti-parallel PIN diodes neutralize the loop in a global view, which is also called intrinsic decoupling. In other possible embodiments, the at least one PIN diode may allow adding or removing additional capacities or inductivities of the local reception coil element. PIN diodes allow current to pass once a certain voltage is applied, meaning, in this case, that a voltage surpassing such a switching voltage is induced into the local reception coil element by the magnetic resonance excitation field (B1 field). When receiving, however, induced voltages are low and the at least one diode does not allow current to pass.

In embodiments, the local reception coil element may have a maximum dimension of 2 to 5 cm. Local reception coil elements of this size are able to couple inductively to external reception coils, in particular a body coil of the magnetic resonance imaging device. Additionally, the depth of penetration of the local reception coil element, that is, the extension of the imaging region into the patient, corresponds essentially to the diameter of the local reception coil element.

Of course, the vascular access device may have additional components, as known from the state of the art. In particular, the vascular access device may further comprise a mandrin and/or a protective cap.

In the case of a mandrin, it may be preferred if the mandrin comprises a wing having an additional local reception coil element. For example, such an additional local coil reception element may be used to obtain magnetic resonance signals during placement of the vascular access device, while the mandrin is still in place inside the vascular cannula and in particular the catheter portion.

It is, additionally, noted that inductively coupling the local reception coil element to an external reception coil usually only reduces the SNR by about 50 % while using the local reception coil element, compared to using only a body coil, increases the SNR by about a factor of 5, so that using the inductive coupling is acceptable.

The invention also comprises a method for tracking a medical instrument in a body region of a patient, the medical instrument being inserted through a vascular cannula of a vascular access device according to the invention, wherein magnetic resonance signals are received by the local reception coil element and used by a magnetic resonance imaging device to reconstruct an image of the body region. All features and comments regarding the vascular access device may correspondingly be applied to the method according to the invention.

In particular, the local reception coil element may be inductively coupled to an external reception coil of the magnetic resonance imaging device, such that the location of the local reception coil element appears bright in the magnetic resonance data of the external reception coil. Enlarging this "brighter" image area yields high quality image data of an imaging region of the local reception coil element.

In concrete embodiments, the body region from which magnetic resonance signals are received by the local reception coil element, that is, the imaging region of the local reception coil element, and from which a corresponding magnetic resonance image may be reconstructed, has at least the length of a mandrin of the vascular access device plus 5 mm parallel to a projection of the mandrin onto the skin surface and/or at least a length of 25 mm perpendicular thereto along the skin surface and/or at least the penetration depth plus 15 mm perpendicular to the skin surface. In this manner, for example, the correct insertion of a guide wire may be tracked.

Further details and advantages of the current invention can be taken from the following description of preferred embodiments in conjunction with the drawings, in which
- Fig. 1: is a schematic drawing of a vascular access device according to the invention,
- Fig. 2: is an equivalent circuit diagram of a first embodiment of a local reception coil element, and
- Fig. 3: is an equivalent circuit diagram of a second embodiment of a local reception coil element.

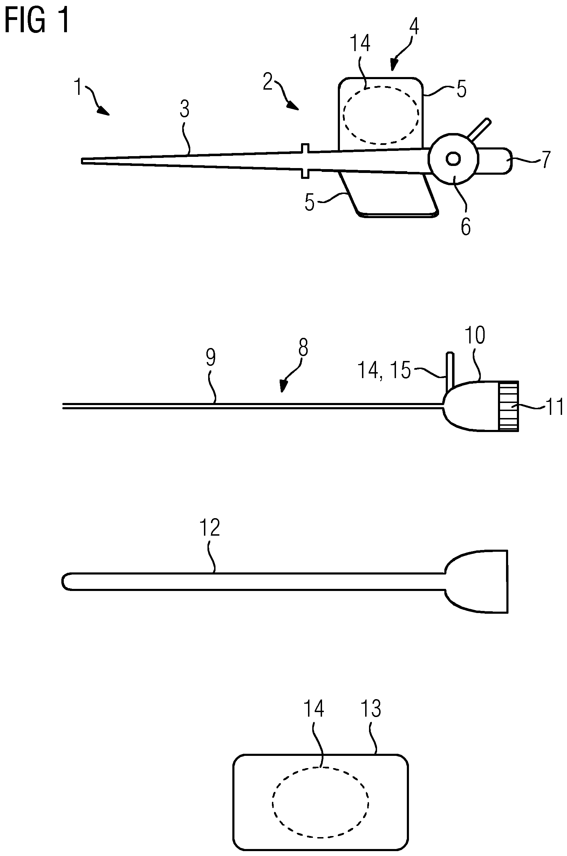

Fig. 1 is a schematic drawing of a vascular access device 1 according to the current invention. As an essential component, the vascular access device 1 comprises a vascular cannula 2, which has a catheter portion 3 whose tip is inserted into a blood vessel, in particular a vein, and a fixing portion 4, in this case having two wings 5, for fixing the vascular cannula 2 onto the skin of a patient as soon as the catheter portion 3 is in place. The vascular cannula 2 may additionally comprise a valve 6 to allow injection of medication with a syringe, and an end 7 to which an intravascular infusion line can be connected and/or through which a medical instrument, for example a guide wire for a therapeutic or treatment catheter, may be inserted into the vascular cannula 2 and therefore the blood vessel.

The vascular access device 1 also comprises a mandrin 8 having a hollow steel portion 9, a blood container 10 and a cap 11. Usually, the mandrin 8 is inserted in the vascular cannula 2 such that the front end of the hollow steel portion 9 is used as a needle to penetrate the skin of a patient and find the suitable blood vessel, wherein insertion into the blood vessel is indicated by blood filling the blood container 10.

The vascular access device 1 may additionally comprise a protective cap 12 and/or a plaster 13 for fixing a wing 5 to the skin of a patient after the vascular cannula 2 and in particular the catheter portion 3 is in place.
According to the invention, at least one wing 5 of the vascular cannula 2 and/or a plaster 13 is provided with at least one local reception coil element 14 for magnetic resonance imaging. It is noted that the mandrin 8 may also have a wing 15 which may have a local reception coil element 14 attached to it or integrated into it.

It is noted at this point that the vascular cannula 2, the blood container 10, the wing 15 and the cap 11 as well as the protective cap 12 are, in this embodiment, all made of plastic.

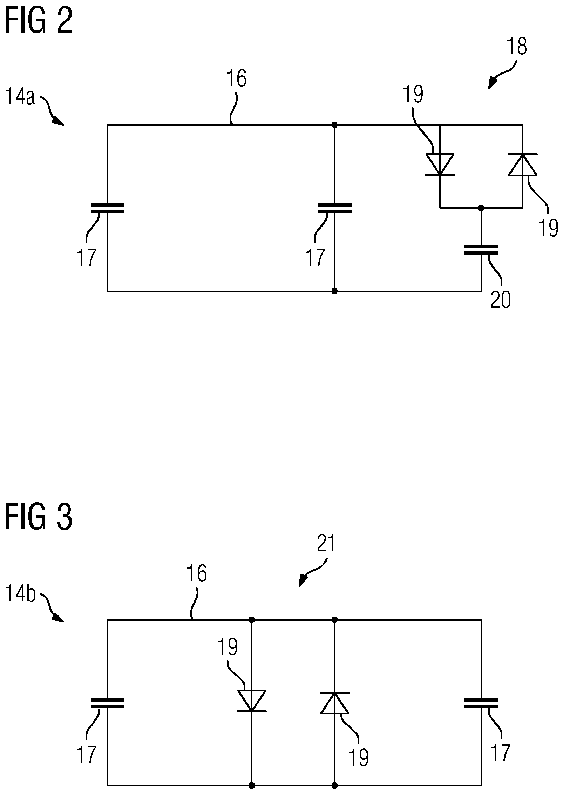

The local reception coil element 14 may, in a less preferred embodiment, resemble a local coil, having a pre-amplifier, a detuning circuit and/or an adaptation circuit. The vascular cannula 2 may then have a connecting device with a cable for the local reception coil element. It is, however, preferred and realized in the shown embodiments that the at least one local reception coil element 14 is inductively coupled to an external reception coil of a used magnetic resonance imaging device, as further explained below with respect to figs. 2 and 3.

While it is conceivable to provide the local reception coil element 14 on a circuit board, in particular a flexible circuit board, it is preferred to print or integrate, in particular by molding, the local reception coil element 14 directly onto or into, respectively, the wing 5 and possibly 15 and/or the plaster 13, wherein preferred embodiments have the local reception coil element 14 at least in one of the wings 5.

As the penetration depths from which magnetic resonance signals may be received by the local reception coil element 14 essentially corresponds to its dimensions, a maximum dimension, in particular a diameter of the loop, of the local reception coil element 14 may preferably be 3 to 5 cm. Such dimensions also allow inductive coupling to the external reception coil.

To enable inductive coupling to the external reception coil of a magnetic resonance imaging device, in particular a local coil and/or a body coil, the local reception coil element 14 comprises a self-resonant structure and/or circuit. In the case of a self-resonant conductor structure, overlapping conductors provide capacitance, while the conductors themselves act as inductances. In a self-resonant circuit, discrete electronic components can be used, for example SMD capacitors.

Fig. 2 is an equivalent circuit diagram of a first embodiment of a local reception coil element 14a. The local reception coil element 14a comprises a primary loop 16 for receiving magnetic resonance signals, in this case realized as a self-resonant conductor structure such that only the capacitors 17 created by overlapping conductors are shown. Inductivities, as explained, are created by the conductors themselves.

The local reception coil element 14a further comprises a detuning device 18 having two PIN diodes 19 acting as switching elements which are provided anti-parallel. As a further detuning element, an additional capacitor 20 is provided.

As the diodes 19 allow current to pass once a certain voltage is surpassed, self-resonance during the transmitting phase of the magnetic resonance imaging device causes one of the diodes 19 to allow current to pass such that the additional capacitor 20 is added and the main loop 16 is detuned, preventing excessive increase of the local B1 field. That is, during the transmitting phase, the local reception coil element 14a is decoupled from the transmitting coil via the frequency shift. In the case of signal reception, the low induced voltages do not suffice to allow current to pass through the diodes 19.

Fig. 3 shows an alternative embodiment of a local reception coil element 14b, in which the detuning device 21 comprises, again, two anti-parallel PIN diodes 19 in the center of the loop 16. In this case, when the voltage limit is surpassed and the diodes 19 allow current to pass due to an applied B1 field in a transmitting phase of the magnetic resonance imaging device, the loop 16 is essentially divided into two sub-loops neutralizing the loop 16 globally by intrinsic decoupling.

The local reception coil element 14, 14a, 14b of the vascular access device 1 can be used to provide magnetic resonance image data of high SNR from an imaging region, in particular covering the whole access site. Such image data can be evaluated to assess correct positioning of the vascular access device 1 and/or track and/or guide insertion of a medical instrument, in particular a guide wire, into the patient using the vascular access device.

Although the present invention has been described in detail with reference to the preferred embodiment, the present invention is not limited by the disclosed examples from which the skilled person is able to derive other variations without departing from the scope of the invention.

## Claims

1. Vascular access device (1), comprising a vascular cannula (2) having a catheter portion (3) for insertion into a blood vessel, in particular a vein, of a patient and a fixing portion (4) for fixing the vascular cannula (2) to a skin of a patient, **characterized in that** the fixing portion (4) and/or a plaster (13) used to fix the fixing portion (4) to the skin comprises at least one local reception coil element (14, 14a, 14b) for receiving magnetic resonance signals.

2. Vascular access device (1) according to claim 1, **characterized in that** the fixing portion (4) comprises at least one wing (5) on or integrated into which the local reception coil element (14, 14a, 14b) is positioned.

3. Vascular access device (1) according to claim 1 or 2, **characterized in that** the local reception coil element (14, 14a, 14b) is printed onto the plaster (13) and/or the fixing portion (4), in particular the wing, or that the local reception coil element (14, 14a, 14b) is provided on a flexible circuit board attached to the plaster (13) and/or fixing portion (4).

4. Vascular access device (1) according to one of the preceding claims, **characterized in that** the local reception coil element (14, 14a, 14b), in particular the whole vascular access device (1), is configured for one-time use.

5. Vascular access device (1) according to one of the preceding claims, **characterized in that** it further comprises at least one connecting device, in particular having a cable, for the local reception coil element (14, 14a, 14b), and/or the local reception coil element (14, 14a, 14b) comprises a pre-amplifier and/or at least one adaptation and/or detuning circuit.

6. Vascular access device (1) according to one of the claims 1 to 4, **characterized in that** the local reception coil element (14, 14a, 14b) is configured to inductively couple to an external reception coil, in particular a body coil and/or an external local coil.

7. Vascular access device (1) according to claim 6, **characterized in that** the local reception coil element (14, 14a, 14b) comprises a self-resonant conducting structure and/or circuit.

8. Vascular access device (1) according to claim 7, **characterized in that** the local reception coil element (14, 14a, 14b) further comprises at least one detuning element for detuning the local reception coil element (14, 14a, 14b) during a magnetic resonance imaging transmitting phase.

9. Vascular access device (1) according to claim 8, **characterized in that** the at least one detuning element comprises at least one switching element, in particular a diode (19), which closes on induction of a predefined voltage into the local reception coil element (14, 14a, 14b) .

10. Vascular access device (1) according to one of the preceding claims, **characterized in that** the local reception coil element (14, 14a, 14b) has a maximum dimension of 2 to 5 cm.

11. Vascular access device (1) according to one of the preceding claims, **characterized in that** it further comprises a mandrin (8).

12. Vascular access device (1) according to claim 11, **characterized in that** the mandrin (8) comprises a wing (15) having an additional local reception coil element (14, 14a, 14b).

13. A method for tracking a medical instrument in a body region of a patient, the medical instrument being inserted through a vascular cannula (2) of a vascular access device (1) according to one of the preceding claims, wherein magnetic resonance signals are received by the local reception coil element (14, 14a, 14b) and used by a magnetic resonance imaging device to reconstruct an image of the body region.

14. Method according to claim 13, **characterized in that** the body region from which magnetic resonance signals are received by the local reception coil element (14, 14a, 14b) and reconstructed has at least the length of a mandrin (8) of the vascular access device (1) plus 5 mm parallel to a projection of the mandrin (8) onto the skin surface and/or at least a length of 25 mm perpendicular thereto along the skin surface and/or at least the penetration depth plus 15 mm perpendicular to the skin surface.

## Patentansprüche

1. Vorrichtung für vaskulären Zugriff (1), umfassend eine vaskuläre Kanüle (2) mit einem Katheterabschnitt (3) zum Einführen in ein Blutgefäß, insbesondere eine Vene, eines Patienten und einem Fixierabschnitt (4) zum Fixieren der vaskulären Kanüle (2) an einer Haut eines Patienten, **dadurch gekennzeichnet, dass** der Fixierabschnitt (4) und/oder ein Pflaster (13), das zum Fixieren des Fixierabschnitts (4) an der Haut verwendet wird, mindestens ein lokales Empfangsspulenelement (14, 14a, 14b) zum Empfangen von Magnetresonanzsignalen umfasst.

2. Vorrichtung für vaskulären Zugriff (1) nach Anspruch 1, **dadurch gekennzeichnet, dass** der Fixierabschnitt (4) mindestens einen Flügel (5) umfasst, worauf oder worin integriert das lokale Empfangsspulenelement (14, 14a, 14b) positioniert ist.

3. Vorrichtung für vaskulären Zugriff (1) nach Anspruch 1 oder 2, **dadurch gekennzeichnet, dass** das lokale Empfangsspulenelement (14, 14a, 14b) auf das Pflaster (13) und/oder den Fixierabschnitt (4) gedruckt ist, insbesondere auf den Flügel, oder dass das lokale Empfangsspulenelement (14, 14a, 14b) auf einer flexiblen Schaltplatine bereitgestellt wird, die an dem Pflaster (13) und/oder Fixierabschnitt (4) befestigt ist.

4. Vorrichtung für vaskulären Zugriff (1) nach einem der vorhergehenden Ansprüche, **dadurch gekennzeichnet, dass** das lokale Empfangsspulenelement (14, 14a, 14b), insbesondere die gesamte Vorrichtung für vaskulären Zugriff (1), zum einmaligen Gebrauch ausgelegt ist.

5. Vorrichtung für vaskulären Zugriff (1) nach einem der vorhergehenden Ansprüche, **dadurch gekennzeichnet, dass** sie des Weiteren mindestens eine Verbindungsvorrichtung, insbesondere mit einem Kabel, für das lokale Empfangsspulenelement (14, 14a, 14b) umfasst und/oder das lokale Empfangsspulenelement (14, 14a, 14b) einen Vorverstärker und/oder mindestens eine Adaptierungs- und/oder Verstimmschaltung umfasst.

6. Vorrichtung für vaskulären Zugriff (1) nach einem der Ansprüche 1 bis 4, **dadurch gekennzeichnet, dass** das lokale Empfangsspulenelement (14, 14a, 14b) ausgelegt ist, um induktiv an eine externe Empfangsspule zu koppeln, insbesondere eine Körperspule und/oder eine externe lokale Spule.

7. Vorrichtung für vaskulären Zugriff (1) nach Anspruch 6, **dadurch gekennzeichnet, dass** das lokale Empfangsspulenelement (14, 14a, 14b) eine selbstresonante leitfähige Struktur und/oder Schaltung umfasst.

8. Vorrichtung für vaskulären Zugriff (1) nach Anspruch 7, **dadurch gekennzeichnet, dass** das lokale Empfangsspulenelement (14, 14a, 14b) des Weiteren mindestens ein Verstimmelement zum Verstimmen des lokalen Empfangsspulenelements (14, 14a, 14b) während einer Übertragungsphase der Magnetresonanzbildgebung umfasst.

9. Vorrichtung für vaskulären Zugriff (1) nach Anspruch 8, **dadurch gekennzeichnet, dass** das mindestens eine Verstimmelement mindestens ein Schaltelement umfasst, insbesondere eine Diode (19), die bei Induktion einer vordefinierten Spannung in das lokale Empfangsspulenelement (14, 14a, 14b) sperrt.

10. Vorrichtung für vaskulären Zugriff (1) nach einem der vorhergehenden Ansprüche, **dadurch gekennzeichnet, dass** das lokale Empfangsspulenelement (14, 14a, 14b) eine Maximalabmessung von 2 bis 5 cm aufweist.

11. Vorrichtung für vaskulären Zugriff (1) nach einem der vorhergehenden Ansprüche, **dadurch gekennzeichnet, dass** sie ferner einen Mandrin (8) umfasst.

12. Vorrichtung für vaskulären Zugriff (1) nach Anspruch 11, **dadurch gekennzeichnet, dass** der Mandrin (8) einen Flügel (15) mit einem zusätzlichen lokalen Empfangsspulenelement (14, 14a, 14b) umfasst.

13. Verfahren zur Verfolgung eines medizinischen Instruments in einer Körperregion eines Patienten, wobei das medizinische Instrument durch eine vaskuläre Kanüle (2) einer Vorrichtung zum vaskulären Zugriff (1) nach einem der vorhergehenden Anspruche eingeführt wird, wobei Magnetresonanzsignale von dem lokalen Empfangsspulenelement (14, 14a, 14b) empfangen und von einer Magnetresonanzbildgebungsvorrichtung verwendet werden, um ein Bild der Körperregion zu rekonstruieren.

14. Verfahren nach Anspruch 13, **dadurch gekennzeichnet, dass** die Körperregion, von der Magnetresonanzsignale durch das lokale Empfangsspulenelement (14, 14a, 14b) empfangen und rekonstruiert werden, mindestens die Länge eines Mandrins (8) der Vorrichtung für vaskulären Zugriff (1) plus 5 mm parallel zu einer Projektion des Mandrins (8) auf der Hautoberfläche und/oder mindestens eine Länge von 25 mm senkrecht dazu entlang der Hautoberfläche und/oder mindestens die Eindringtiefe plus 15 mm senkrecht zu der Hautoberfläche aufweist.

## Revendications

1. Dispositif (1) d'accès vasculaire, comprenant une canule (2) vasculaire ayant une partie (3) cathéter pour l'insertion dans un vaisseau sanguin, en particulier une veine, d'un patient et une partie (4) de fixation pour fixer la canule (2) vasculaire à la peau d'un patient, **caractérisé en ce que** la partie (4) de fixation et/ou un sparadrap (13) utilisé pour fixer la partie (4) de fixation à la peau, comprend au moins un élément (14, 14a, 14b) local de bobine de réception pour recevoir des signaux de résonnance magnétique.

2. Dispositif (1) d'accès vasculaire selon la revendication 1, **caractérisé en ce que** la partie (4) de fixation comprend au moins une aile sur ou intégrée dans laquelle l'élément (14, 14a, 14b) local de bobine de réception est mis en position.

3. Dispositif (1) d'accès vasculaire selon la revendication 1 ou 2, **caractérisé en ce que** l'élément (14, 14a, 14b) local de bobine de réception est imprimé sur le sparadrap (13) et/ou la partie (4) de fixation, en particulier l'aile, ou **en ce que** l'élément (14, 14a, 14b) local de bobine de réception est prévu sur une plaquette à circuit imprimé souple fixée au sparadrap (13) et/ou à la partie (4) de fixation.

4. Dispositif (1) d'accès vasculaire selon l'une des revendications précédentes, **caractérisé en ce que** l'élément (14, 14a, 14b) local de bobine de réception, en particulier le dispositif (1) d'accès vasculaire tout entier, est configuré pour être utilisé une seule fois.

5. Dispositif (1) d'accès vasculaire selon l'une des revendications précédentes, **caractérisé en ce qu'**il comprend en outre au moins un dispositif de connexion, ayant en particulier un câble, pour l'élément (14, 14a, 14b) local de bobine de réception, et/ou l'élément (14, 14a, 14b) local de bobine de réception comprend un préamplificateur et au moins un circuit d'adaptation et/ou de désaccord.

6. Dispositif (1) d'accès vasculaire selon l'une des revendications 1 à 4, **caractérisé en ce que** l'élément (14, 14a, 14b) local de bobine de réception est configuré pour se coupler inductivement à une bobine de réception extérieure, en particulier une bobine de corps et/ou une bobine extérieure locale.

7. Dispositif (1) d'accès vasculaire selon la revendication 6, **caractérisé en ce que** l'élément (14, 14a, 14b) local de bobine de réception comprend une structure et/ou un circuit conducteur auto-résonnant.

8. Dispositif (1) d'accès vasculaire selon la revendication 7, **caractérisé en ce que** l'élément (14, 14a, 14b) local de bobine de réception comprend en outre au moins un élément de désaccord pour désaccorder l'élément (14, 14a, 14b) local de bobine de réception pendant une phase de transmission d'imagerie à résonnance magnétique.

9. Dispositif (1) d'accès vasculaire selon la revendication 8, **caractérisé en ce que** au moins un élément de désaccord comprend au moins un élément d'interruption, en particulier une diode (19), qui se ferme à l'induction d'une tension définie préalablement à l'élément (14, 14a, 14b) local de bobine de réception.

10. Dispositif (1) d'accès vasculaire selon l'une des revendications précédentes, **caractérisé en ce que** l'élément (14, 14a, 14b) local de bobine de réception a une dimension maximum de 2 à 5 cm.

11. Dispositif (1) d'accès vasculaire selon l'une des revendications précédentes, **caractérisé en ce qu'**il comprend en outre un mandrin (8).

12. Dispositif (1) d'accès vasculaire selon la revendication 11, **caractérisé en ce que** le mandrin (8) comprend une aile (15) ayant un élément (14, 14a, 14b) local supplémentaire de bobine de réception.

13. Procédé pour suivre un instrument médical dans une partie du corps d'un patient, l'instrument médical étant inséré par une canule (2) vasculaire d'un dispositif (1) d'accès vasculaire selon l'une des revendications précédentes, dans lequel des signaux de résonnance magnétique sont reçus par l'élément (14, 14a, 14b) local de bobine de réception et utilisés par un dispositif d'imagerie à résonnance magnétique pour reconstruire une image de la partie du corps.

14. Procédé selon la revendication 13, **caractérisé en ce que** la partie du corps dont les signaux de résonnance magnétique sont reçus par l'élément (14, 14a, 14b) local de bobine de réception et qui est reconstruite a au moins la longueur d'un mandrin (8) du dispositif (1) d'accès vasculaire plus 5 mm parallèlement à une projection du mandrin (8) sur la surface de la peau et/ou au moins une longueur de 25 mm perpendiculairement à celle-ci le long de la surface de la peau et/ou au moins la profondeur de pénétration plus 15 mm perpendiculairement à la surface de la peau.
